# EUROPEAN PATENT SPECIFICATION

(11) **EP 0 720 463 B1**
(45) Date of publication and mention of the grant of the patent: **30.12.1998**
(21) Application number: 94928634.8
(22) Date of filing: 20.09.1994
(51) Int. Cl.: A61F 13/15, A61F 13/58

(54) **ABSORBENT ARTICLES HAVING UNDERGARMENT COVERING COMPONENTS WITH PATTERNED REGIONS OF EXTENSIBILITY**
ABSORBIERENDE ARTIKEL MIT WÄSCHESCHUTZELEMENTEN MIT STRUKTURIERTEN, DEHNBAREN REGIONEN
ARTICLES ABSORBANTS DOTES D'ELEMENTS DE RECOUVREMENT DE SOUS-VETEMENTS PRESENTANT DES REGIONS CONFIGUREES D'EXTENSION

(30) Priority: 20.09.1993 US 124180; 19.09.1994 US 308188
(43) Date of publication of application: 10.07.1996
(73) Proprietor: THE PROCTER & GAMBLE COMPANY, Cincinnati, Ohio 45202 (US)
(72) Inventor: HAMMONS, John, Lee, Hamilton, OH 45011 (US); COE, Richard, George, Cincinnati, OH 45244 (US); DeHAAN, Dennis, Allen, Cincinnati, OH 45231 (US); WEINGERGER, Eric, Patton, Fairfield, OH 45014 (US); ALDRICH, Ronald, John, Cincinnati, OH 45251 (US); MANSFIELD, Michel Ann, Apartment 2, Cincinnati, OH 45220 (US)
(74) Representative: Hirsch, Uwe Thomas
(86) International application number: US9410652
(87) International publication number: WO9508311

(56) References cited:
- EP-A- 0 337 438
- WO-A-92/07528
- WO-A-93/06805
- US-A- 4 940 462
- US-A- 5 098 755

## Description

### FIELD OF THE INVENTION

The present invention relates to absorbent articles such as sanitary napkins, panty liners, and incontinence pads. More particularly, the present invention relates to sanitary napkins that have undergarment covering components (or "side wrapping elements") that are provided with a pattern of regions of extensibility, which automatically wrap the sides of a wearer's undergarments when the undergarments are pulled up to provide an alternative to conventional side flaps.

### BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perineal area of the body. Sanitary napkins both with and without side flaps (or wings) are disclosed in the literature and are available in the marketplace.

Generally when sanitary napkins are provided with flaps the flaps extend laterally from a central absorbent means and are intended to be folded around the edges of the wearer's panties in the crotch region. Commonly, the flaps are provided with an attachment means for either affixing the flaps to the underside of the wearer's panties or to the opposing flap. The flaps are particularly effective for preventing exudates from soiling the edges of the wearer's panties.

Sanitary napkins having flaps of various types are disclosed in U.S. Patent 4,687,478, entitled "Shaped Sanitary Napkin With Flaps" which issued to Van Tilburg on August 18, 1987; U.S. Patent 4,608,047, entitled "Sanitary Napkin Attachment Means", which issued to Mattingly on August 26, 1986; U.S. Patent 4,589,876, entitled "Sanitary Napkin", which issued to Van Tilburg on May 20, 1986 and its Reexamination Patent No. B1 4,589,876, Certificate of Reexamination issued April 27, 1993; U.S. Patent 4,285,343, entitled "Sanitary Napkin", which issued to McNair on August 25, 1981; U.S. Patent 3,397,697, entitled "Disposable Sanitary Shield For Undergarments", which issued to Rickard on August 20, 1968; and, U.S. Patent 2,787,271, entitled "Sanitary Napkin", which issued to Clark on April 2, 1957.

While sanitary napkins having flaps are commonly viewed as providing better protection against soiling as compared to sanitary napkins without flaps, some women find applying sanitary napkins having flaps to be inconvenient for various reasons. For instance, some women find it to be difficult to attach the flaps to the underside of the crotch of their panties. This can be due to factors such as the tendency for the adhesive fasteners on the flaps to stick to themselves or to other parts of the sanitary napkin. As a result, some women still prefer a sanitary napkin without flaps. In addition, some women who generally prefer a sanitary napkin with flaps, occasionally (such as during periods of light flow) prefer a sanitary napkin without flaps. Therefore, there is a need for a sanitary napkin which provides an alternative to sanitary napkins having conventional side flaps while still providing the protection of side flaps.

Several variations of sanitary napkins having conventional flaps that attempt to solve some, but not all of these problems are disclosed in the patent literature. For example, U.S. Patent 4,911,701 issued to Mavinkurve discloses a sanitary napkin having elastic strands for providing a greater convex shape to the body-facing portion of the central absorbent and for enabling adhesive-free placement of the flaps of a winged napkin embodiment into a pair of panties. The sanitary napkin described in the Mavinkurve patent, however, still appears to require the user to manipulate the flaps (by first flipping the flaps upward and then placing the sanitary napkin in her panties and flipping the flaps back down) since the flaps appear to be predisposed to be in a downward folded condition The Mavinkurve patent also requires that individual elastic strands be attached in a contracted condition to the central absorbent portion of the napkin and/or to its wings or flaps The napkins described in the Mavinkurve patent can, therefore, be difficult and expensive to manufacture U.S. Patent 4,940,462 issued to Salerno discloses a sanitary napkin with longitudinally expandable flaps. The flaps are designed to fold over the exterior of the wearer's panty and then to expand longitudinally to conform with the contour of the panties. The Salerno patent, however, appears to require conventional adhesive fasteners to retain the flaps in place on the underside of the wearer's panties.

WO 93/06805 discloses sanitary napkins with flaps having zones of differential extensibility. This disclosure suggests to use different zones of differential extensibility to achieve a quantitative variation of extensibility in the side flaps. No consideration is given to a variation in direction of extensibility.

Thus, a need exists for an absorbent article, such as a sanitary napkin, that is provided with an alternative to conventional flaps. In particular, a need exists for a sanitary napkin having an alternative to conventional flaps which provides the protection from soiling of conventional flaps and which can conveniently and efficiently solve the problems caused when attempting to attach conventional flaps to the underside of the wearer's panties.

It is, therefore, an object of the present invention to provide an absorbent article, such as a sanitary napkin, that is able to provide coverage to the wearer's panties to reduce side soiling (i.e., staining of the edges of the panty crotch) without the use of conventional flaps.

It is another object of the present invention to provide an absorbent article, such as a sanitary napkin, that automatically wraps around the sides of the wearer's panties by the simple action of the wearer pulling up her panties.

It is still another object of the present invention to provide an absorbent article, such as a sanitary napkin, that is able to wrap around the sides of the wearer's panties and stay without providing flaps having panty fasteners thereon, and without attaching separate elastic strands to the sanitary napkin.

These and other objects of the present invention will be more readily apparent when considered in reference to the following description and when taken in conjunction with the accompanying drawings.

### SUMMARY OF THE INVENTION

The present invention provides an absorbent article, such as a sanitary napkin. The sanitary napkin of the present invention has a pair of undergarment covering components (or "side wrapping elements") with patterned regions of extensibility. The side wrapping elements automatically wrap the side edges of the wearer's panties to provide coverage to the wearer's panties to reduce side soiling (i.e., staining of the edges of the panty crotch) without the use of conventional flaps.

The sanitary napkin comprises a main body portion comprising a liquid pervious topsheet, a liquid impervious backsheet joined to the topsheet, and an absorbent core positioned between the topsheet and the backsheet. The side wrapping elements are preferably joined to, and more preferably, integral with the main body portion. The side wrapping elements extend laterally outward beyond the longitudinal side edges of the main body portion a short distance beyond the crotch edge portions of the wearer's undergarment. The side wrapping elements are provided with a pattern of regions of extensibility. The pattern of regions of extensibility comprises at least a first region that has a primary component of extensibility in a first direction and a second region that has a primary component of extensibility in a second direction. The components of extensibility of the regions are oriented in different directions. Numerous patterns of regions of extensibility are possible. The patterned regions of extensibility can be used to provide the side wrapping elements with particular folding characteristics.

The sanitary napkin of the present invention provides an alternative to sanitary napkins having conventional side flaps for several reasons. The side wrapping elements do not extend far enough outward beyond the side edges of the wearer's panties to cause any inconvenience to the wearer. The side wrapping elements require no action on the part of the wearer to fold the side wrapping elements under her panties or to attach the same to her panties. The side wrapping elements stay in place well enough to cover the sides edges of the wearer's panties without affixing them underneath the wearer's panties.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:
FIG. 1 is a top plan view of an embodiment of the sanitary napkin of the present invention that has a first region of transverse extensibility along the proximal edge of the side wrapping elements and a second region of longitudinal extensibility which is located outboard of the first region.
FIG. 2 is a side view of the sanitary napkin shown in FIG. 1.
FIG. 3 is an end view of the sanitary napkin shown in FIG. 1 shown with a portion of the topsheet cut away to show the absorbent core.
FIG. 4 is a top plan view of an alternative embodiment of the sanitary napkin of the present invention that has a continuum of regions of extensibility in which there is a first region along the transverse centerline of the side wrapping elements which is longitudinally extensible, and a plurality of second regions located longitudinally outboard of the first region that have components of extensibility that angle away from the longitudinal centerline at progressively greater angles.
FIGS. 5 and 6 are top plan views of variations of the embodiment shown in FIG. 4 in which the first and second regions comprise discrete zones of extensibility.
FIG. 7 is a top plan view of an alternative embodiment of the sanitary napkin of the present invention which has side wrapping elements that are defined by a strainable network with curvilinear bands of less extensibility.
FIG. 7A is a top plan view of a polymeric web material having a simplified version of the strainable network which is used in the side wrapping elements of the sanitary napkin shown in FIG. 7.
FIGS. 7B-D are enlarged segmented perspective illustrations of the polymeric web material shown in FIG. 7A in which the web material moves from an untensioned condition to progressively greater tensioned conditions.
FIGS. 8-10 are top plan views of alternative embodiments of the sanitary napkin of the present invention which has side wrapping elements with corrugations in which the fold lines of the corrugations radiate outward in several different patterns.
FIG. 11 is a plan view of an alternative embodiment of a sanitary napkin having side wrapping elements with a less extensible band therein.
FIG. 12 is a schematic perspective view of the sanitary napkin shown in FIG. 11 showing the manner in which the less extensible band maintains the side wrapping element in a folded condition around the side of the wearer's panty.
FIG. 13 is a perspective view of a portion of a side wrapping element that has regions near the distal edge with deeper corrugations than the regions near the proximal edge.
FIG. 14 is an exaggerated schematic side view of a pair of mating plates that can be used to provide the corrugations in the side wrapping element shown in FIG. 13.
FIG. 15 is a sectional view of the engagement of the teeth of the mating plates shown in FIG. 13 taken along line 15-15 of FIG. 13.
FIG. 16 is a sectional view of the engagement of the teeth of the mating plates shown in FIG. 13 taken along line 16-16 of FIG. 13.
FIG. 17 is a plan view of a sanitary napkin which shows the manner in which the patterned regions can provide the sanitary napkin with the appearance of a particular shape.
FIG. 18 is an enlarged schematic view of a portion of the side wrapping element of an alternative embodiment of the sanitary napkin with regions of extensibility that are in a staggered pattern.
FIG. 19 is a schematic side view of the distal portion of a side wrapping element which shows a way the components of the side wrapping elements can be folded to provide improved resistance to edge compression.
FIG. 20 is a schematic side view of a side wrapping element which is formed of a structure that provides the side wrapping element with additional bulk for improved resistance to edge compression.

### DETAILED DESCRIPTION OF THE INVENTION

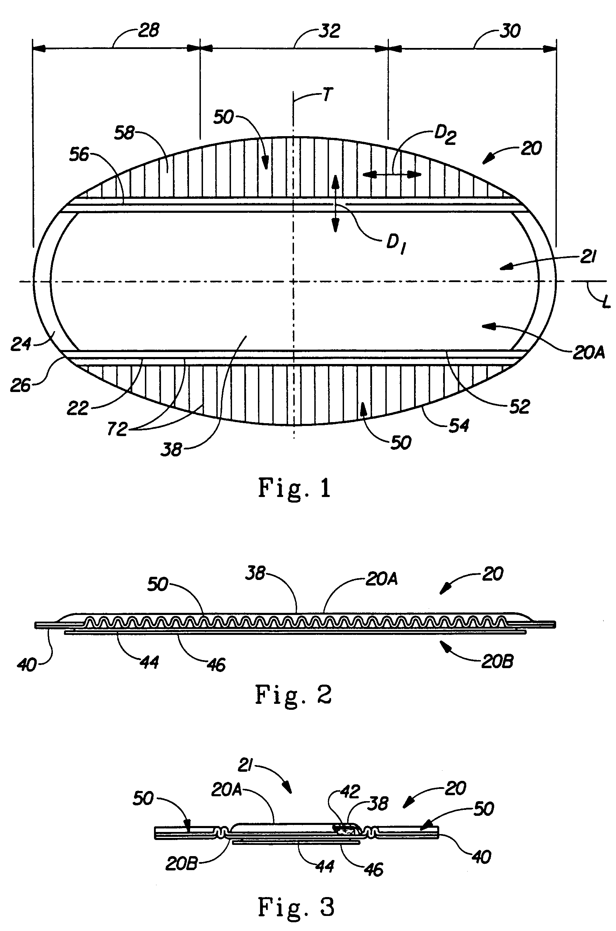

FIGS. 1-3 show one preferred embodiment of a disposable absorbent article of the present invention 20. The present invention relates to absorbent articles, such as sanitary napkins, panty liners, and incontinence pads. More particularly, the present invention relates to sanitary napkins that have a main body portion 21 and a pair of side wrapping elements 50 that automatically wrap the sides of the wearer's panties when the wearer places the sanitary napkin in her panties and pulls her panties up adjacent to her body.

The sanitary napkin 20 has two surfaces, a liquid pervious body-contacting surface or "body surface" 20A and a liquid impervious garment surface 20B. The sanitary napkin 20 is shown in FIG. 1 as viewed from its body surface 20A. The body surface 20A is intended to be worn adjacent to the body of the wearer. The garment surface 20B of the sanitary napkin 20 (shown in FIG. 2) is intended to be placed adjacent to the wearer's undergarments when the sanitary napkin 20 is worn.

The sanitary napkin 20 has two centerlines, a longitudinal centerline L and a transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 1 shows that the main body portion 21 of the sanitary napkin 20 comprises the portion of the sanitary napkin other than the side wrapping elements. The main body portion 21 has two spaced apart longitudinal edges 22, two spaced apart transverse or end edges (or "ends") 24, which together form the periphery 26 of the main body portion. The main body portion also has two end regions, which are designated first end region 28 and second end region 30. A central region 32 is disposed between the end regions 28 and 30. The end regions 28 and 30 extend outwardly from the edges of the central region 32 about 1/8 to about 1/3 of the length of the main body portion. A detailed description of a sanitary napkin having a central region 32 and the two end regions 28 and 30 is contained in U.S. Patent 4,690,680 issued to Higgins on September 1, 1987.

The main body portion 21 of the sanitary napkin 20 can be of any thickness, including relatively thick, relatively thin, or even very thin. The embodiment of the sanitary napkin 20 shown in Figures 1-3 of the drawings is intended to be an example of a relatively thin sanitary napkin, preferably an "ultra-thin" sanitary napkin as described in U.S. Patents 4,950,264 and 5,009,653 issued to Osborn. The sanitary napkin 20 shown should preferably also be relatively flexible, so that it is comfortable for the wearer. It should be understood that the sanitary napkin shown in FIGS. 1-3 is merely one preferred embodiment of the present invention, and that the present invention is not limited to absorbent articles of the type or having the specific configurations shown in the drawings.

FIG. 3 shows the individual components of the main body portion 21 of the sanitary napkin 20 of the present invention. The main body portion 21 shown in FIG. 3 generally comprises at least three primary components. These include a liquid pervious topsheet 38, a liquid impervious backsheet 40, and an absorbent core 42 positioned between the topsheet 38 and the backsheet 40. There may be occasions, however, when one or more of these components, such as the backsheet, can be replaced by a component that serves as part of the side wrapping elements 50 described herein. The topsheet, the backsheet, and the absorbent core may be assembled in a variety of configurations known in the art (including so called "sandwich" products and "tube" products).

Several preferred sanitary napkin configurations are described generally in the following patents and patent application: U.S. Patent 4,321,924, "Bordered Disposable Absorbent Article" issued to Ahr on March 30, 1982, U.S. Patent 4,425,130, "Compound Sanitary Napkin" issued to DesMarais on January 10, 1984; U.S. Patent 4,950,264, "Thin, Flexible Sanitary Napkin" issued to Osborn on August 21, 1990; U.S. Patent 5,308,346, "Elasticized Sanitary Napkin" issued to Sneller, et al. on May 3, 1994; U.S. Patent 5,584,829 entitled "Absorbent Articles Having Panty Covering Components That Naturally Wrap the Sides of Panties" filed July 22, 1993, in the name of Lavash, et al.; and PCT Application WO 95/07675 entitled "Absorbent Articles Having Panty Covering Components Comprising Extensible Web Materials Which Exhibit Elastic-Like Behavior" in the name of Mansfield, et al. The main body portion 21 of the sanitary napkin may also be comprised of one or more extensible components such as the components used in the sanitary napkins, and the like described in PCT Publication Nos. WO 93/01785 and 93/01786, both published February 4, 1993.

Figures 1-3 show a preferred embodiment of the sanitary napkin 20 assembled in a sandwich construction in which the topsheet 38 and the backsheet 40 have length and width dimensions generally larger than those of the absorbent core 42. The topsheet 38 and the backsheet 40 extend beyond the edges of the absorbent core 42 to form portions of the periphery 26 of the main body portion 21. The sanitary napkin 20 shown in FIGS. 1-3 comprises pair of side wrapping elements 50 that extend laterally outward beyond the longitudinal side edges 22 of the main body portion 21 from their proximal edges 52 to their distal edges 54.

The side wrapping elements 50 can be of any suitable size and shape. The side wrapping elements 50 of the present invention may have any of the dimensions set forth for the panty covering components in the aforementioned US 5,584,829 and WO 95107675 Thus, distal edges 54 of the side wrapping elements preferably extend outward beyond the longitudinal side edges 22 of the main body portion 21, a distance of less than one-half the width of the main body portion.

The side wrapping elements 50 can be made from any of the materials used in the construction of the main body portion 21 of the sanitary napkin. The side wrapping elements 50 preferably comprise materials that provide enough resistance to edge compression so that the side wrapping elements will fold, rather than crumple, when they are subjected to compression by the wearer's thighs. These components can include absorbent components if the components of the side wrapping elements are suitably sealed to prevent wicking of exudates from the distal edges of the side wrapping elements. Suitable anti-wicking crimp seals are described in U.S. Patent 4,321,924 issued to Ahr. The components of the side wrapping elements that form the garment-facing side of the side wrapping elements should also preferably be liquid impervious. In the preferred embodiment shown in FIGS. 1-3, the side wrapping elements 50 comprise a three layer laminate. From top to bottom, the laminate comprise an extensible nonwoven web for comfort, an extension of the topsheet comprising an apertured formed film made in accordance with U.S. Patent 4,342,314 issued to Radel, et al. and U.S. Patent 4,463,045 issued to Ahr, et al., and an extension of the backsheet which comprises a polyethylene film.

The side wrapping elements 50 can be joined to the main body portion 21 in any suitable manner. The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element. Thus, the side wrapping elements 50 can be integral extensions of the topsheet 38 and backsheet 40 of the main body portion 21. This is the situation in the embodiment shown in FIGS. 1-3. In other embodiments, the side wrapping elements 50 may comprise separate elements that are joined to the main body portion. In such embodiments, the side wrapping elements 50 can, for example, comprise two separate components, or a single component (which may be referred to as a "panty covering component" or "undergarment covering component"). In still other embodiments, each side wrapping element 50 can comprise more than one component.

The side wrapping elements 50 (or at least one thereof) comprises an element such as a web of material or a laminate. The element preferably has at least two regions which have primary components of extensibility that are oriented in different directions. The term "primary component of extensibility", as used herein, refers to a vector component that is oriented in the direction of the predominant extensibility of the region. The side wrapping elements 50 preferably, each comprise at least a first region 56 that has a primary component of extensibility in a first direction D₁, and a second region 58 that has a primary component of extensibility in a second direction D₂. However, other variations are also possible.

The different regions of the side wrapping elements 50 can be extensible primarily in the longitudinal direction (that is, more extensible in the longitudinal direction than the transverse direction), primarily in the transverse direction (that is, more extensible in the transverse direction than in the longitudinal direction), or in any direction between the longitudinal direction and the transverse direction. The side wrapping elements 50 can have extensible portions distributed across their entire surface, or they can have regions of extensibility distributed only on limited areas within of the side wrapping elements 50. The side wrapping elements 50 may, thus, be said to have a pattern of extensibility. This pattern of extensibility comprises various portions or regions of extensibility. The term "pattern of extensibility" as used herein, means that the sanitary napkin has at least two regions of extensibility as described herein. The side wrapping elements 50 may, but need not actually have a visible pattern therein as shown in FIG. 1.

The regions of extensibility can be discrete regions of the side wrapping elements in which there is a line of demarcation that separates one region from another. In other embodiments, the regions can comprise a continuum of extensibility in which there is no line of demarcation between the different regions of extensibility. A virtually unlimited number of embodiments of the present invention are possible. For example, the side wrapping elements 50 of the invention can have a plurality of first and second regions of extensibility. The side wrapping elements 50 can also have additional regions of extensibility that are extensible in other directions (that is, other than in the first and second directions). These additional regions of extensibility can be referred to herein as third, fourth, fifth, . . ., etc. regions of extensibility. If desired, however, the third, fourth, fifth, . . . etc. regions of extensibility may be viewed together as portions of the second region of extensibility for simplicity. (This may be desirable for ease of describing a structure if there are a large number of regions of extensibility that have distinct regions of extensibility, but vary only slightly between regions.) In still other embodiments, the side wrapping elements 50 may have regions having extensibility in a particular direction that are located within other regions that are extensible in a different direction.

The embodiments shown in the drawings will now be examined in greater detail. The embodiment shown in FIGS. 1-3 has a first region 56 that is disposed along the proximal edge 52 of the side wrapping elements 50. The first region 56 has a primary component of extensibility that runs in a direction D₁, which is oriented primarily in the transverse direction. The second regions 58 are disposed laterally outboard of the first regions 56. The second regions 58 in this embodiment extend from the edge of the first regions 56 to the distal edges 54 of the side wrapping elements and extend the entire length of the side wrapping elements. The second regions 58 have a primary component of extensibility that runs in a direction D₂, which is oriented more in the longitudinal direction than the primary component of extensibility of the first region 56. Preferably, in this embodiment the primary component of extensibility of the second regions 58 is oriented primarily in the longitudinal direction.

The advantage of providing the side wrapping elements 50 with transverse or lateral extensibility in the first regions 56 is that the extensibility can be used to disassociate (or "decouple" any forces, such as those that cause bunching of the main body portion 21 of the sanitary napkin from affecting the wrapping function of the side wrapping elements 50. These forces can be caused by the application of laterally-inward oriented compressive forces by the wearer's thighs and/or by the elasticized edges of the wearer's panties.

In the embodiment shown in FIGS. 1-3, the first region 56 comprises a narrow longitudinally-oriented region (preferably about 6 mm wide) with corrugations (e.g., alternating ridges and valleys) having longitudinally-oriented fold lines therein. The second regions 58 comprise wider longitudinally-oriented regions with corrugations having transversely-oriented fold lines therein. The extensibility of such corrugated structures is generally perpendicular to the fold lines.

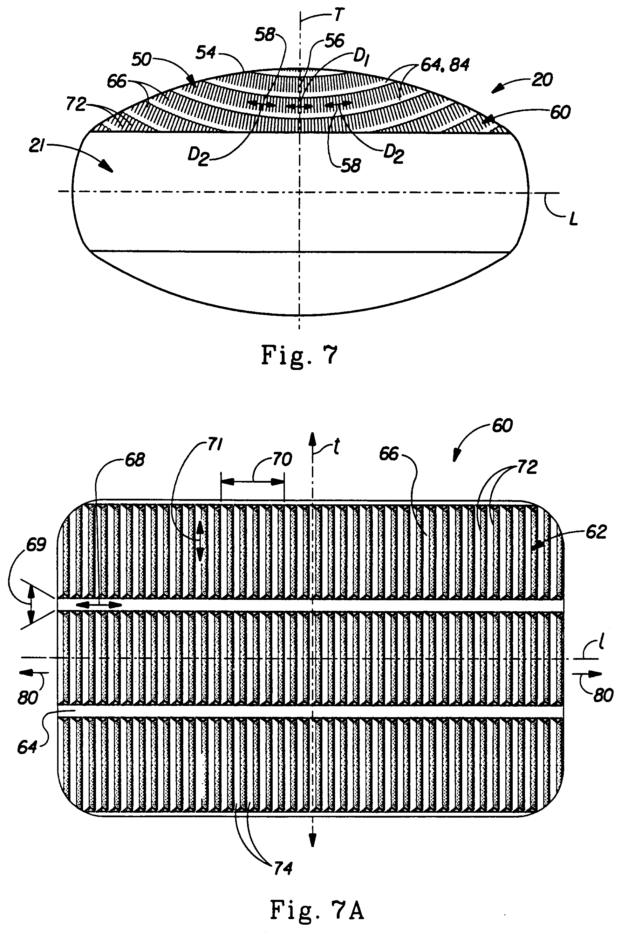

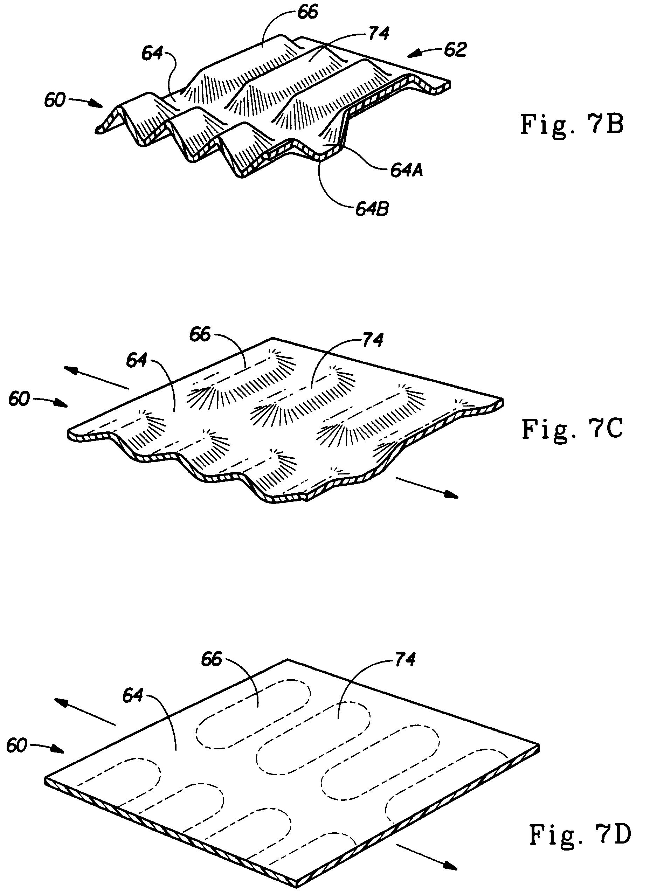

The side wrapping elements 50 are preferably provided with the two regions of extensibility by either ring rolling (or pre-corrugating), and or by forming strainable networks in the different regions of the side wrapping elements 50 such as by subjecting portions of the side wrapping elements 50 to compression between the mating plates. Suitable methods for ring rolling are described in U.S. Patent 4,107,364 issued to Sisson on August 15, 1978, U.S. Patent 4,834,741 issued to Sabee on May 30, 1989, U.S. Patent 5,143,679 issued to Gerald M. Weber, et al. on September 1, 1992, U.S. Patent 5,156,793 issued to Kenneth B. Buell, et al. on October 20, 1992, and U.S. Patent 5,167,897 issued to Gerald M. Weber, et al. on December 1, 1992. The formation of a strainable network into a web material is described below in conjunction with the embodiment shown in FIG. 7. In alternative versions of this embodiment any other suitable method of providing the various regions of extensibility can be used. For example, the different regions of extensibility can be provided by joining together one or more extensible materials in a quilt-like fashion so that the various patches of extensible material are oriented so that their extensibility is in different directions. Ring rolling and the process of forming a strainable network in a material are particularly preferred for this purpose, however, because such operations can be readily adapted for use in high speed manufacturing operations. Further, the process of forming a strainable network in a material is highly preferred because it can be adapted to produce a virtually unlimited number of patterns.

The side wrapping elements 50 preferably have a sufficient resistance to edge compression so that they will fold rather than crumple when placed in a wearer's panties and subjected to the forces associated with wearing the sanitary napkin. The term "resistance to edge compression" refers to how substantial the material that comprises the side wrapping elements 50 is. Specifically, edge compression refers to the tendency of the side wrapping elements 50 to buckle when the side wrapping elements are extended to form a planar extension and forces are applied to the distal edge 54 of the side wrapping element by a plate oriented perpendicular to the plane of the side wrapping elements 50. This property is important because if the side wrapping elements 50 are insubstantial, they will bunch up when forces are applied to the side wrapping elements by the wearer's panty elastics or by the wearer's thighs during wear. The side wrapping elements 50 should, of course, not be so stiff that they are uncomfortable to the wearer.

The garment surface 20B of the sanitary napkin 20 may include, and preferably does include, fasteners for attaching the sanitary napkin to the wearer's undergarments. Figure 2 shows the central pad fastener 44 which is adapted to secure the main body portion 21 of the sanitary napkin to the crotch region of an undergarment. Fasteners comprising adhesives have been found to work well for this purpose, with pressure sensitive adhesives being preferred. The adhesive fastener is typically covered with a removable cover strip or release liner 46 in order to keep the adhesive from sticking to a surface other than the crotch portion of the panty prior to use. Suitable release liners are also described in the above-referenced U.S. Patent 4,917,697.

The sanitary napkin 20 of the present invention is used by removing any release liner 46 and placing the sanitary napkin 20 in a panty so that the adhesive (or other fastener) 44 contacts the panty and maintains the sanitary napkin in position within the panty during use. The side wrapping elements 50 automatically wrap around the sides of the wearer's panty by the simple action of the wearer pulling up her panties.

The operation of the side wrapping elements 50 is distinguishable in several respects from that of conventional side flaps. Placing a sanitary napkin having conventional flaps in a pair of panties and pulling up the panties will not consistently provide the automatic sustained wraparound feature of the present invention. There are several reasons for this. Conventional flaps are not provided with resistance to edge compression so that they will tend to crumple in use, particularly when the wearers thighs exert compressive forces on the flaps. Conventional flaps are also not provided with zones of extensibility, so they will generally not wrap around and conform to the panties. In those cases where conventional flaps do wrap around the panties, they will not consistently stay wrapped since conventional flaps do not have zones of extensibility. Further, conventionally-sized flaps will have excess flap material that hangs down underneath the panties during wear. This material can move around excessively underneath the panties and be uncomfortable for the wearer. The side wrapping elements of the present invention, on the other hand have a span that is ideally just wide enough to wrap around the elastic-containing edges of the panties, but no wider, avoiding the problems associated with excess flap material. The side wrapping elements 50 can be provided with still other features.

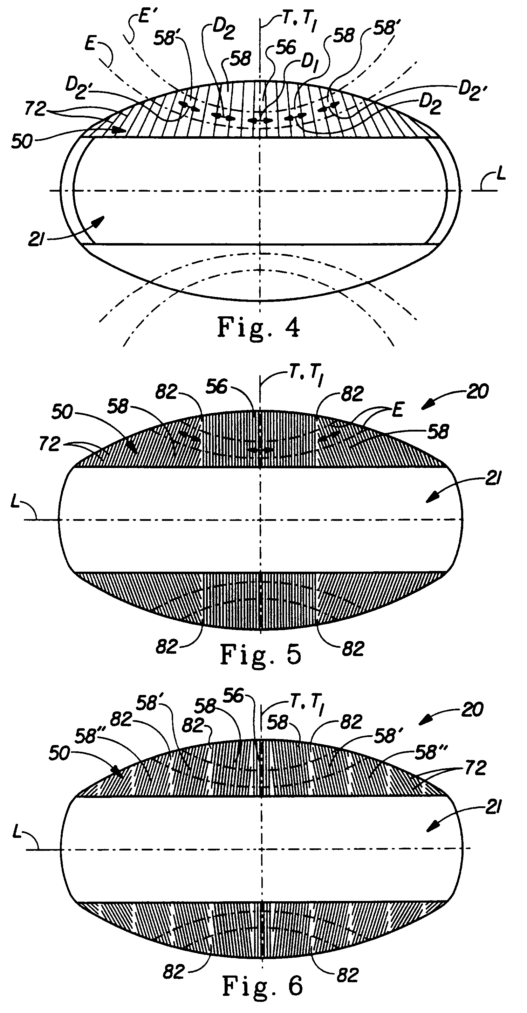

FIG. 4 shows a second embodiment of the present invention. In the embodiment shown in FIG. 4, the first region 56 comprises a region that includes the transverse centerline T₁ of the side wrapping elements 50. The primary component of extensibility of the first region, D₁, is oriented primarily in the longitudinal direction. The side wrapping element 50 comprises a plurality of second regions that are located longitudinally outboard of the first region 56 on both sides of the first region 56. Since there is more than one second region, these are designated as 58 and 58' for purposes of illustration. The second regions 58 and 58' form an uninterrupted continuum of extensible regions that have primary components of extensibility that angle away from the longitudinal centerline L. The second regions 58 and 58' on each side of the first region 56 have primary components of extensibility D₂ and D₂ʼ , respectively. The primary components of extensibility D₂ and D₂ʼ form progressively greater angles away from the longitudinal centerline of the sanitary napkin the further the second regions 58 are spaced from the first region 56. The extensibility of the embodiment shown in FIG. 4 is established so that the fold lines 72 of the corrugations are oriented approximately at a 90° angle to the wearers panty elastic. The dashed lines, E and E', in FIG. 4 represent the elastic configurations of a range of panty styles. The orientation of the fold lines in the different regions of the side wrapping elements makes the various regions of the side wrapping elements 50 extensible in the same direction as the panty elastics. This embodiment has the advantage of providing extensibility in each portion of the side wrapping elements that closely matches that needed to conform to the stretching of the wearer's panty elastics.

FIGS. 5 and 6 show examples of sanitary napkins having side wrapping elements 50 with zones of extensibility in other directions and in alternative arrangements. In the examples shown in FIGS. 5 and 6, instead of being provided with first and second regions which define an uninterrupted continuum of extensibility as in the case of FIG. 4, the first and second regions 56 and 58 are located in discrete regions. These regions are separated by laterally-oriented less extensible regions 82. FIG. 5 shows an embodiment comprising three regions of extensibility, a first region 56, and a second region 58 on both sides of the first region 56. FIG. 6 shows an embodiment which comprises a plurality of discrete second regions, 58, 58', 58", etc., located on both sides of the first region 56.

FIG. 7 shows an example of a sanitary napkin similar to that of FIG. 4, only having side wrapping elements 50 with regions of extensibility that have at least one, and preferably more than one, generally longitudinally-oriented band 84 of less extensibility therein. The bands 84 of less extensibility can be linear, curvilinear (as shown in FIG. 7), or partially linear and partially curvilinear. The bands 84 of less extensibility, if curvilinear, can be concave relative to the longitudinal centerline L as shown in FIG. 7, or they can be convex relative to the longitudinal centerline The curvilinear bands 84 of less extensibility provide potential folding axes about which the side wrapping elements 50 may fold. This allows the side wrapping elements 50 to fold smoothly around the curved edges of a pair of panties. The multiple bands 84 allow the side wrapping elements 50 to fold around a variety of panty sizes.

The pattern of extensibility in the side wrapping elements 50 shown in FIG. 7 is preferably provided by a strainable network that is formed in the side wrapping elements 50. More particularly, the web material (or laminate) comprising the side wrapping elements 50 in FIG. 7 has a strainable network of regions formed therein. This strainable network is especially useful because it exhibits an elastic-like behavior in the direction of elongation without the use of added elastic materials. FIGS. 7A-7D show enlarged views of a web material having a strainable network formed therein. FIGS. 7A and 7B depict simplified versions of the strainable web material 60 shown in FIG. 7. The versions shown in FIGS. 7A and 7B are simplified in that the less extensible regions are straight rather than curved as shown in FIG. 7. FIGS. 7A and 7B show the strainable web material 60 in an untensioned condition. The strainable web material 60 has a longitudinal centerline (or axis), l, and a lateral centerline (or axis), t. The longitudinal centerline, l, can be rectilinear, curvilinear, or partially both.

FIGS. 7A and 7B show that the strainable web material 60 includes a strainable network 62 of at least two distinct and dissimilar regions. The term "strainable network", as used herein, refers to an interconnected and interrelated group of regions which are able to be extended to some useful degree in a predetermined direction. The two distinct regions provide the strainable web material 60 with a first elastic-like, relatively low resistive force stage and a second relatively high resistive force stage. The strainable network 62 is formed into the strainable web material 60. As used herein, the term "formed" refers to the creation of a desired structure or geometry upon the web material that will substantially retain the desired structure or geometry when it is not subjected to any externally applied elongations or forces. Suitable methods for forming a material such as the strainable web material include, but are not limited to embossing by mating plates or rolls, thermoforming, high pressure hydraulic forming, or casting.

FIGS. 7A and 7B show that the two distinct regions of the strainable network 62 include at least a first region 64 and a second region 66. (It should be noted that the first and second regions 64 and 66 of the strainable network 62 are distinguishable from the first and second regions 56 and 58 of extensibility of the side wrapping elements 50. The regions of the strainable network 62 may, however, be related to the first and second regions 56 and 58 of extensibility of the side wrapping elements 50. The two sets of regions may be related to each other in a number of different ways. For instance, as shown in FIG. 7, the first and second regions 56 and 58 of extensibility of the side wrapping elements 50 may both be a part of second regions 66 of the strainable web material. The curvilinear bands 84 of less extensibility in the embodiment shown in FIG. 7 are provided by the first regions 64 of the extensible web material.)

The strainable network 60 is configured so that the first region 64 will exhibit resistive forces in response to an applied axial elongation in a direction parallel to a predetermined axis before a substantial portion of the second region 66 develops significant resistive forces to the applied elongation. The first and second regions of the strainable network each have a first surface and an opposing surface. The configuration of the material having a strainable network is described in terms the surface-pathlengths of the different regions. The first region 64 has a surface-pathlength which is less than that of the second region 66. The surface-pathlengths are measured substantially parallel to the predetermined axis while the material is in an untensioned condition. The second region 66 includes one or more deformations 74 which extend beyond the plane of the first region 64.

In the preferred embodiment shown in FIGS. 7A and 7B, the strainable network 62 includes a plurality of first regions 64 and a plurality of second regions 66. In the preferred embodiment shown in FIGS. 7A and 7B, the first regions 64 are substantially planar regions. That is, the material within the first region 64 is in substantially the same condition before and after the formation step undergone by strainable web material. The second regions 66 include a plurality of continuous, interconnected, deformations 74 which extend alternately beyond the plane of both the first and second surfaces (64A and 64B, respectively) of the first region 64. In other embodiments, the deformations 74 may extend beyond the plane of only one of either the first or the second surfaces of the first region.

The first regions 64 of the strainable network 62 have a first axis 68 and a second axis 69, wherein the first axis 68 is preferably longer than the second axis 69. In the simplified embodiment shown, the first axis 68 of the first region 64 is substantially parallel to the longitudinal axis, l, of the strainable web material 60 while the second axis 69 is substantially parallel to the transverse axis, t, of the strainable web material 60. The second regions 66 of the strainable network also have a first axis 70 and a second axis 71. The first axis 70 of the second region 66 is substantially parallel to the longitudinal axis l of the strainable web material 60, while the second axis 71 is substantially parallel to the transverse axis t of the strainable web material 60. In the simplified version of the strainable web material shown in FIGS. 7A and 7B, the first regions 64 and the second regions 66 are substantially linear, extending continuously in a direction substantially parallel to the longitudinal axis l of the strainable web material. In the sanitary napkin embodiment shown in FIG. 7, the first regions 64 of the strainable network are curvilinear and the second regions 66 are linear and perpendicular to the first regions 64. In the embodiment shown in FIG. 7, the longitudinal centerline l of the strainable web material 60 is generally curvilinear. The longitudinal centerline l of the strainable web material 60 is generally oriented in the longitudinal direction. In other embodiments, however, the longitudinal centerline l of the web material can be oriented in other directions, depending on the direction of extensibility desired.

The strainable web material 60 preferably comprises at least one component which is a formed polymeric film. The strainable web material 60 can be made of a base material that has a relatively low extensibility under the forces the sanitary napkin is normally subjected to when worn. When formed into the strainable web material 60 as described herein, however, the base material will be extensible under the forces the sanitary napkin is normally subjected to when worn. The strainable web material 60 is preferably comprised substantially of linear low density polyethylene (LLDPE). The strainable web material 60 may also be comprised of other polyolefins such as polyethylenes, including low density polyethylene (LDPE). ultra low density polyethylene (ULDPE), high density polyethylene (HDPE), or polypropylene and blends thereof with the above and other materials. Examples of other suitable polymeric materials which may also be used include, but are not limited to polyester, polyurethanes, compostable or biodegradable polymers, heat shrink polymers, thermoplastic elastomers, and breathable polymeric structures. Strainable web materials suitable for the present invention are described in greater detail in the aforementioned WO 95107675

FIGS. 7B, C and D show the manner in which the strainable web material 60 exhibits at least two significantly different stages of controlled resistive force to elongation when subjected to an applied elongation in a direction parallel to a predetermined axis. The strainable web material 60 exhibits first resistive forces to the applied elongation (which develop between the stage shown in FIG. 7B and the stage shown in FIG. 7C). The first resistive forces occur until the elongation of the web is sufficient to cause a substantial portion of the second region 66 to enter the plane of applied elongation, as shown in FIG. 7C. After the strainable web material 60 reaches the stage shown in FIG. 7C, it exhibits second resistive forces to further elongation (as illustrated by FIG. 7D). Typically, when used in the side wrapping elements 50 of the present invention, the web material will be within the first stage of resistance to elongation so the various portions of the strainable web material 60 will only extend to the stage shown in FIG. 7C and adjust so as to relax back to the stage shown in FIG. 7B.

The depth and number of deformations 74 in the strainable web material 60 can be varied to control the applied force or elongation required to extend the web material used in the side wrapping elements 50. In one preferred embodiment, the deformations 74 are formed by two rigid plates having outer dimension of 127 mm by 304.8 mm by 19.05 mm (5.0" by 12" by 0.75"). On one surface of each plate are a series of meshing teeth which are substantially triangular in cross section and measure 0.762 mm (0.030") at their bases and taper to a vertex with a radius 0.2032 mm (0.008") at the top. The centerlines of the teeth are spaced evenly and at 0.762 mm (0.030") increments. On the "toothed" side of one plate, a series of grooves are cut which are parallel to each other and perpendicular to the evenly spaced teeth. These grooves measure 0.7874 mm (0.031") wide and are continuous over the entire length of the plate, and are spaced at a distance of 6.35 mm (0.25") on center. These grooves correspond to the undeformed regions of the deformed web of material. The preferred web material is placed between the plates in a hydraulic press having platens larger than the plates to evenly distribute pressure. The plates are compressed under a load of at least 1816 kg (4,000 pounds). The formed web material is then removed from between the plates. The available stretch or elongation is increased if for a given number of deformations, the height or degree of deformation imparted to the web material is increased. Similarly the available stretch or elongation is increased if for a given height or degree of deformation, the number or frequency of deformations is increased.

The above-described method for providing the side wrapping elements 50 with a strainable network is particularly well suited to providing the side wrapping elements 50 with different regions of extensibility. The mating plates can be configured to create any of the patterns of extensibility within the side wrapping elements of the sanitary napkins shown in the drawings. In order to create the patterns shown in some of the drawing figures, this may involve elimination of the first regions 64 of the strainable network (such as inextensible bands 84) from the side wrapping elements 50. The elimination of the first regions 64 has the effect of substantially reducing or eliminating the elastic-like tendency for the various regions of the side wrapping elements to retract (and, thus, the two different stages of controlled resistive force to elongation). In many embodiments, however, reducing the tendency of the various regions of the side wrapping elements to retract is preferred, especially when it is desired to have the side wrapping elements stay wrapped around the sides of the wearer's panty crotch once they are folded around the panties.

The foregoing method is also particularly well-suited for providing different regions of extensibility on sanitary napkins having integral side wrapping elements. Integral designs have the advantage of providing reduced manufacturing complexity and materials cost. The patterned regions of extensibility can be imparted to both of the side wrapping elements of a sanitary napkin simultaneously. This eliminates the need for a method which requires the steps of: cutting pieces for the side wrapping elements; aligning the pieces with the longitudinal edges of the main body portion; and securing the pieces to the main body portion. This can be done by utilizing a pair of rollers or plates that have recessed areas therein for the main body portion if it is only desired to impart a pattern on the side wrapping elements. Alternatively, if it desirable to impart a pattern into the main body portion (such as to provide extensibility therein), the entire "engagement area" of the plates can be provided with a pattern. In this latter case, the depth of the engagement areas on the rollers or plates can be established so as to be spaced closer together or (preferably) farther apart relative to the portions of the plates that will engage the main body portion than over the side wrapping elements to accommodate the difference in caliper of these different portions of the sanitary napkin.

Further, the above-described process may be used in integral designs in a way to optimize the location and amount of extensibility in different regions of the side wrapping elements 50. This allows additional design flexibility compared to a single type of pre-processed uniformly extensible material, since the material properties may be altered along the length of the side wrapping element, optimizing for panty style, product core, pad thickness, or pad length. Several alternative embodiments shown in the figures that follow illustrate this design flexibility.

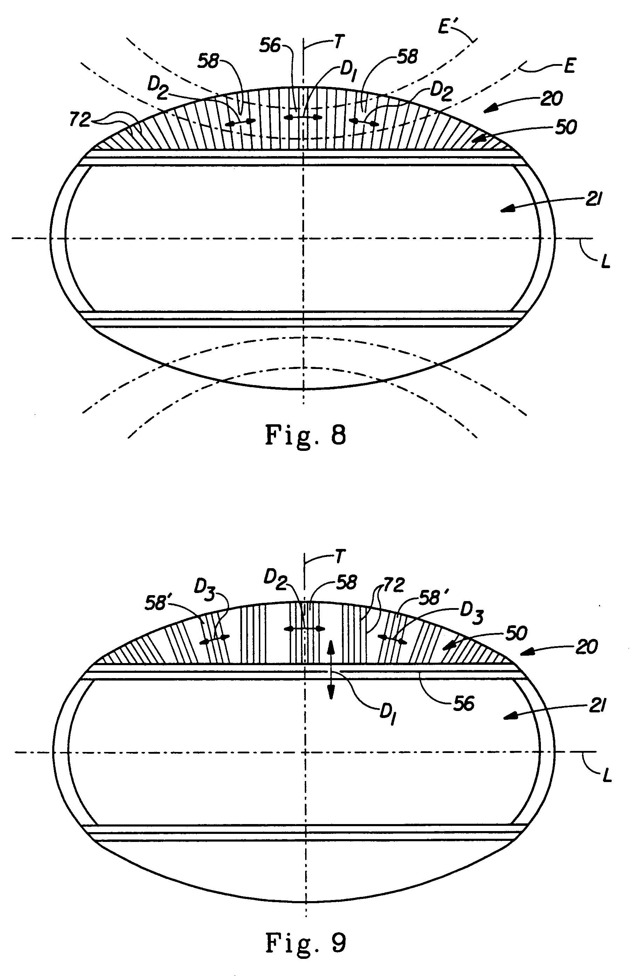

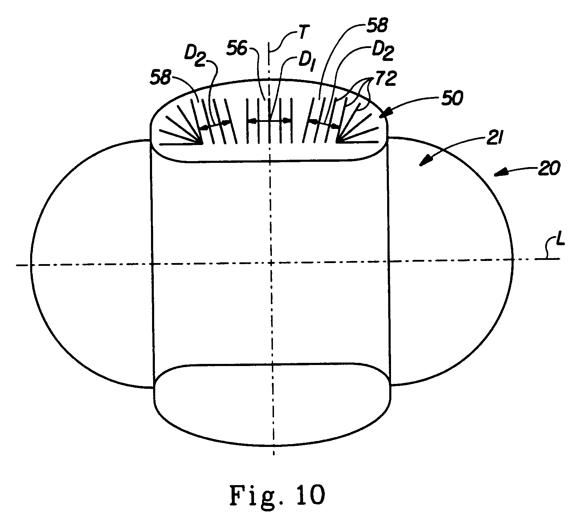

FIGS. 8-10 show examples of sanitary napkins having side wrapping elements 50 with regions of extensibility in which the corrugation lines 72 of the extensible regions of the side wrapping elements 50 radiate outward. FIGS. 8-10 also provide examples of embodiments in which the direction of the components of extensibility D₂ of the second regions 58 angle progressively more toward (rather than away from) the longitudinal centerline L of the sanitary napkin to create "fan-like" structures. FIG. 8 shows an example of a sanitary napkin in which the different regions of extensibility form an uninterrupted continuum of extensibility. FIG. 9 provides an example of a sanitary napkin with side wrapping elements having a "hybrid" pattern of extensibility. The sanitary napkin shown in FIG. 9 has a combination of the characteristics of the regions of extensibility shown in several of the other drawing figures (e.g., similar to the structures shown in FIG. 1 and in FIG. 8 only in discrete spaced apart regions. FIG. 10 shows an example of a structure in which the corrugation fines 72 of the side wrapping elements radiate outward from several different points. A virtually unlimited number of other patterns are possible.

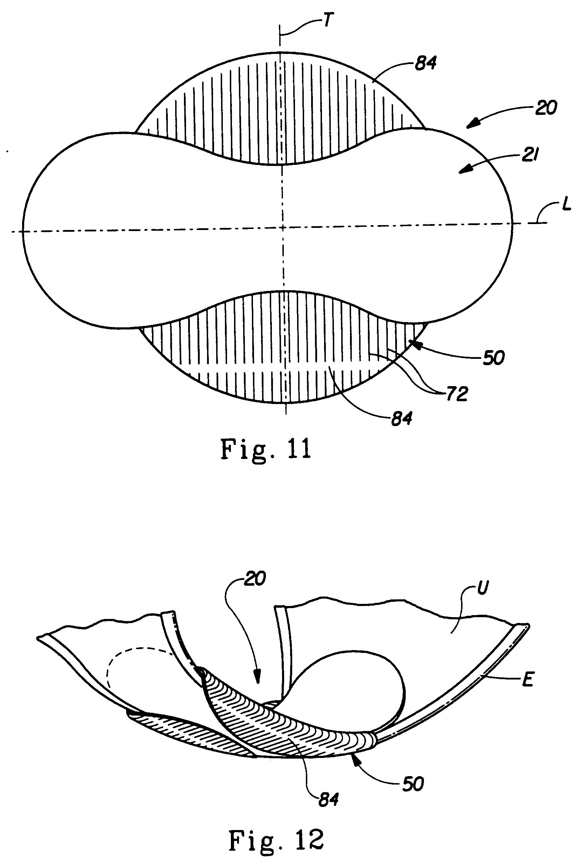

FIGS. 11 and 12 show an alternative embodiment of a sanitary napkin having side wrapping elements 50 with a less extensible band 84 therein. The less extensible band 84 is preferably generally longitudinally oriented. The less extensible band 84 is also preferably spaced far enough laterally outward that it will lie underneath the crotch of the wearer's panties or undergarments, U, when the sanitary napkin 20 is worn. This will allow the less extensible band 84 to maintain a line of tension in the longitudinal direction through at least part of the length of the side wrapping element. As shown in FIG. 12, the line of tension will be maintained because the less extensible band 84 will tend to take the configuration of a straight path from one end to the other while the rest of the side wrapping element 50 will tend to follow the arcuate curvature of the bottom of the wearer's panty crotch. This has the advantage of establishing a force within the side wrapping element 50 for ensuring that the side wrapping element stays in place folded around the side edge of the wearer's panty crotch.

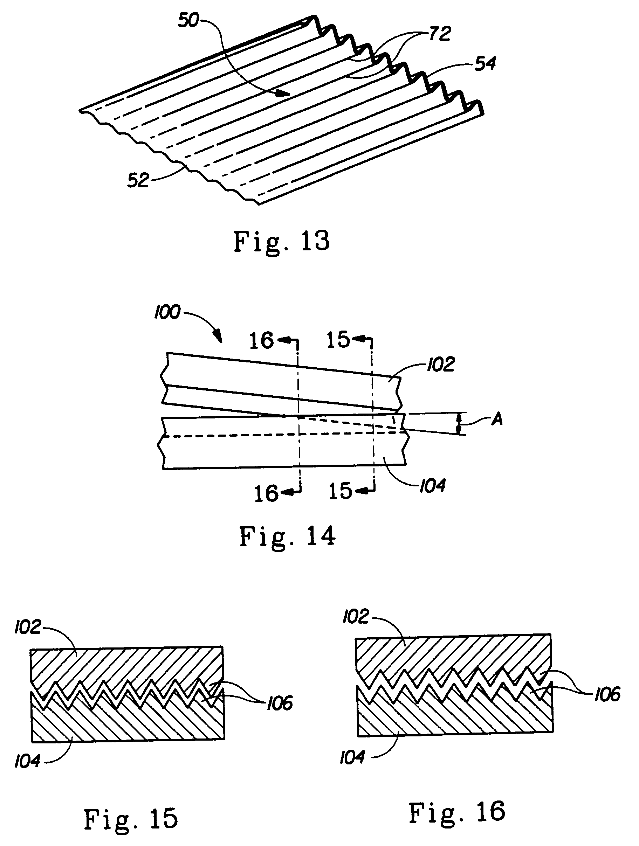

FIG. 13 shows a portion of another alternative side wrapping element embodiment. The side wrapping element in FIG. 13 has progressively deeper corrugations in the regions near its distal edge 54 than in the regions near its proximal edge 52. This side wrapping element 50 provides the advantage that the amount of extensibility is greater toward the distal edge 54 where it is needed the most, and the proximal edge 52 is stiffer to provide improved resistance to edge compression.

FIGS. 14-16 are schematic views of an apparatus 100 that can be used to make the side wrapping element shown in FIG. 13. The apparatus 100 comprises a pair of meshing plates (or rollers) 102 and 104 that have a plurality of teeth 106. FIG. 14 shows (in a somewhat exaggerated fashion) that the plates 102 and 104 are "canted" or tilted at an angle (angle A) relative to each other. The tilting of the plates 102 and 104 causes the space between the teeth 106 of the plates to engage each other in different amounts. FIG. 15 shows that along one cross-section (line 15-15), the teeth of the plates engage each other more fully. This end forms the corrugations at the distal edge 54 of the side wrapping element 50. FIG. 16 shows that the teeth of the plates engage each other less fully near the other end of the plate which will form the proximal edge 52 of the side wrapping element.

Other ways of making a structure that behaves similarly to the side wrapping element 50 shown in FIG. 13 are also possible. For example, the pattern of teeth on the plates or rolls can be modified so that the side wrapping elements have more extensibility near their distal edge. This can be done by utilizing plates that have more closely-spaced teeth, longer teeth, or a larger number of finer teeth on the portions of the plates that will "form" the regions along the distal edge of the side wrapping elements. The use of plates with finer corrugations can also provide the side wrapping elements with the benefit of increased softness along the distal edges of the side wrapping elements.

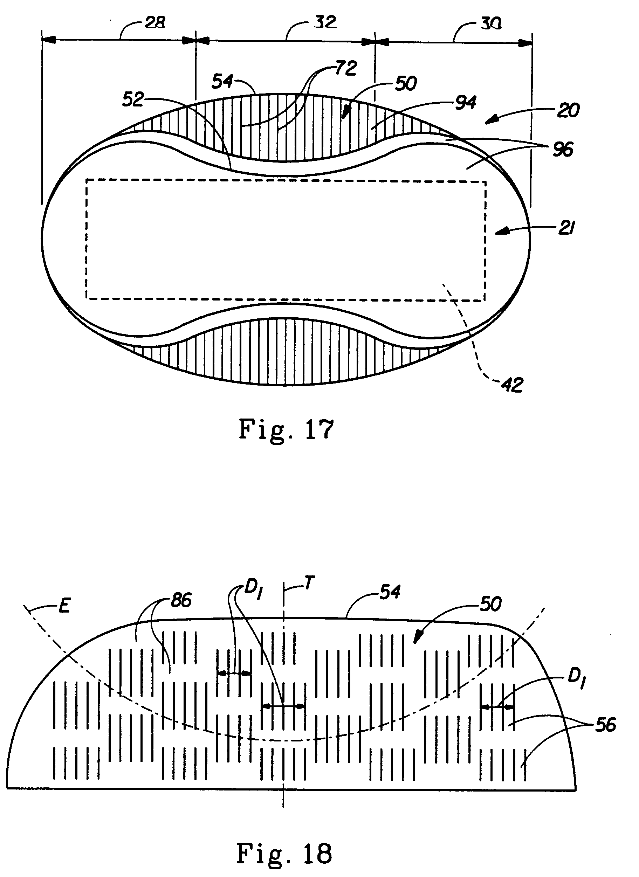

FIG. 17 shows that in other alternative embodiments the patterned regions of extensibility can be used to provide the sanitary napkin with the appearance of a particular shape. The absorbent core 42 of the sanitary napkin shown in FIG 17 is generally rectangular. The sanitary napkin 20 can have a pattern formed therein as shown in FIG. 17 that provides it with the appearance of having an hourglass-shaped main body portion 21.

FIG. 18 shows an embodiment of a sanitary napkin (only a portion of which is shown) having still another different type of pattern of regions of extensibility. The embodiment shown in FIG. 18 has a side wrapping element 50 that comprises a staggered pattern of regions of extensibility. The regions of extensibility shown in FIG. 18 differ from those shown in the previous embodiments in that the regions are in a pattern, but the components of extensibility that are all oriented in the same direction. The embodiment shown in FIG. 18 is useful, however, because the staggering of the regions of extensibility reduces the tendency for the side wrapping elements 50 to crumple and buckle (that is, it provides improved resistance to edge compression). In addition, the side wrapping elements 50 in FIG. 18 can fold about any of the short longitudinally-oriented regions 86 so that the side wrapping elements 50 can adapt to a wide variety of panty sizes and styles.

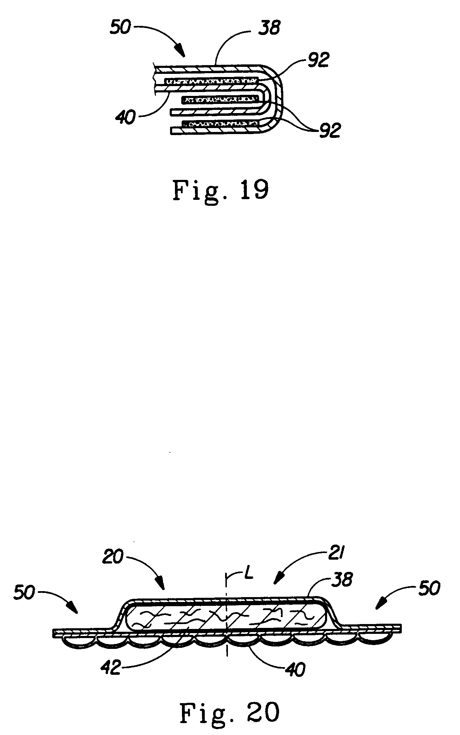

The side wrapping elements 50 can also be provided with improved resistance to edge compression in other ways. Suitable ways of providing the side wrapping elements 50 with the desired resistance to edge compression include, but are not limited to: utilizing stiffer materials; extending another component of the sanitary napkin, such as an acquisition layer, into the side wrapping elements 50; doubling over or otherwise folding at least one of the components of the side wrapping elements to create double or more thicknesses of the same; providing the side wrapping elements with stiffeners, such as elastomeric scrims, and the like; either providing the side wrapping elements with bulking members or forming the side wrapping elements into structures with additional bulk; and, altering the regions or patterns of extensibility in the side wrapping elements (as shown in FIG. 18) Forming the side wrapping elements 50 into structures with additional bulk can be done in a non-limiting number of ways, including using glue, foam, and entrapped air. FIGS. 19 and 20 further illustrate a few additional examples of the above concepts.

FIG. 19 shows one of the ways that the components of the side wrapping elements 50 can be folded to double the thickness of the components. In FIG. 19, both the topsheet 36 and backsheet 40 are folded under and secured to the portions of the topsheet and backsheet that extend from the longitudinal side edges 22 of the main body portion using a strip of adhesive 92. This provides the side wrapping elements 50 with improved resistance to edge compression.

FIG. 20 shows another one of the ways that the side wrapping elements 50 can be formed into structures with additional bulk. In FIG. 20, the additional bulk is provided by utilizing a backsheet made of a "bubble" type packaging material.

Preferably, if other components of the sanitary napkin are extended out into the side wrapping elements, these components are oriented so that any inherent extensibility of such components is in the direction of the predominant extensibility desired in the side wrapping elements. Further, if such components are absorbent, the sanitary napkin will need an anti-wicking crimp seal along the distal edges of the side wrapping elements to prevent liquids from wicking out of the side wrapping elements.

The side wrapping elements 50 can also be provided with a variety of optional additional properties. These include, but are not limited to constructing the side wrapping elements out of materials, or treating such materials to be hydrophobic, hydrophilic, or to have zones that are hydrophilic so that they are hydrophilic only in regions where liquids are likely to be deposited. For instance, the portions 94 of the side wrapping elements shown in FIG. 17 that lie in the central region 32 of the sanitary napkin and adjacent the distal edge 54 of the side wrapping elements can be treated to render them hydrophobic so liquids will not tend to wick outwardly therefrom. The portions 96 of the side wrapping elements in the end regions 28 and 30 and closer to the proximal edge 52 of the side wrapping elements could be rendered hydrophilic so that liquids will run back toward the absorbent core 42.

In still other embodiments, other variations are possible. For example, different topsheet materials such as nonwovens or softer films can be used to make the body-contacting surface of the side wrapping elements more comfortable for the wearer. For instance, the topsheet can comprise a zoned formed film having a zone that covers the main body portion that comprises an apertured formed film made in accordance with U.S. Patents 4,342,314 issued to Radel, et al. and U.S. Patent 4,463,045 issued to Ahr, et al., and the portion of the topsheet that extends into the side wrapping elements can comprise a film that is made in accordance with U.S. Patent 4,629,643 issued to Curro, et al. to provide a cleaner and drier surface in the area of typical liquid deposition and a softer surface in those areas that will come in contact with the wearer's thighs. In other alternative embodiments, the topsheet could comprise a composite structure having a zone that covers the main body portion that comprises an apertured formed film, and zones of nonwoven material forming the body-contacting structures of the side wrapping elements.

There are numerous possible alternative embodiments and variations of the present invention shown in the preceding drawing figures. For example, the side wrapping elements are preferably mirror images of each other, and are symmetrical about the longitudinal centerline. However, it should be understood that the shape and location of the side wrapping elements described herein are those of a preferred embodiment, and other embodiments are also possible. In addition, while the side wrapping elements 50 are shown as extending from each longitudinal edges of the main body portion, in other alternative embodiments, there may only be one side wrapping element extending from one of the edges of the main body portion. The side wrapping elements 50 may be offset along the longitudinal centerline more towards one end edge of the main body portion than the other. In still other embodiments, the side wrapping elements 50 may be separate elements that are joined underneath to the main body portion 21 of the sanitary napkin inboard of the longitudinal side edges 22 of the main body portion. The side wrapping elements 50, in such a case, may be otherwise unattached to the garment-facing side of the main body portion 21 of the sanitary napkin 20 between the points of attachment and the longitudinal side edges 22 of the main body portion.

In other embodiments, the side wrapping elements 50 can be used to provide the sanitary napkin with a "stand-up" cuff feature to further reduce soiling of the wearer's panties. One possible way of providing such a feature is to add a strip of material, such as a nonwoven material, to the top of each of the side wrapping elements. The nonwoven material can be bonded to the side wrapping elements along the distal edge 54 of the side wrapping elements and at the ends of the side wrapping elements. The nonwoven material can be bonded by fusion, adhesive, or any other suitable type of bonds. The inside edge of the strip of nonwoven material is preferably left partially or totally unbonded so that it will be able to move away from the body-facing side of the main body portion. If this nonwoven material is relatively stiff it will tend to be raised upward when the side wrapping elements 50 are folded around the edges of the wearer's panties. The nonwoven material can also be an elastomeric material that is stretched before it is applied to the side wrapping elements 50. This can be used to aid the nonwoven in standing up, and may also be used to cause the sanitary napkin to assume a curved configuration from front to back for better fit.

In addition,. while it is highly preferred that the patterned regions of extensibility of the sanitary napkin be provided in side wrapping elements, it is also possible that providing patterned regions of extensibility on absorbent articles with flaps of various types will benefit such absorbent articles. Absorbent articles having flaps of various types are described in U.S. Patents 4,589,876, issued May 20, 1986. to Van Tilburg and 4,687,478, issued August 18, 1987, to Van Tilburg, U.S. Patent 5,267,992 issued to Van Tilburg on December 7, 1993, U.S. Patent 5,344,416 issued September 6, 1994, to Niihara, U.S. Patent 5,346,486 issued September 13, 1994, to Osborn, et al., and PCT Publication No. WO 93/06805 published April 15, 1993 entitled "Absorbent Article Having Flaps and Zones of Differential Extensibility" filed in the name of Lavash, et al..

The present invention is also applicable to other types of absorbent articles worn in the crotch region of an undergarment such as pantiliners and incontinence articles. The terms "panty liner" or "pantiliner" refer to absorbent articles that are less bulky than sanitary napkins which are generally worn by women between their menstrual periods. Examples of suitable absorbent articles in the form of pantiliners that can be provided with the side wrapping elements with the patterned regions of extensibility described herein are disclosed in U.S. Patent 4,738,676 entitled "Pantiliner" issued to Osborn on April 19, 1988.

The term "incontinence article" refers to pads, undergarments (pads held in place by a suspension system of same type, such as a belt, or the like), inserts for absorbent articles, capacity boosters for absorbent articles, briefs, bed pads, and the like, regardless of whether they are worn by adults or other incontinent persons Examples of suitable incontinence articles that can be provided with the side wrapping elements with the patterned regions of extensibility described herein are disclosed in U.S. Patent 5,300,054 issued to Feist, et al. on April 5, 1994 and U.S. Patent 5,304,161 issued to Noel, et al. April 19, 1994.

While particular embodiments of the present invention have been illustrated and described, it would be obvious to those skilled in the art that various other changes and modifications can be made

## Claims

1. An absorbent article (20) for wearing in a wearer's undergarment that has a crotch region with a pair of side edges said absorbent article (20) having a longitudinal dimension extending in a longitudinal direction (L) and a transverse dimension extending in a transverse direction (T), said absorbent article (20) comprising:
a main body portion (21) comprising an absorbent core, said main body portion (21) having a body-facing side (21A), a garment-facing side (21B) and a pair of longitudinal side edges (22): and
a pair of side wrapping elements (50) for folding around the side edges of the wearer's undergarment, said side wrapping elements (50) joined to and extending laterally outward beyond the longitudinal side edges (22) of said main body portion (21) to distal edges (54), characterized in that at least one of said side wrapping elements (50) is provided a pattern of regions of extensibility comprising at least a first region (56) that has a primary component of extensibility in a first direction (D₁) and a second region (58) that has a primary component of extensibility in a second direction (D₂) which is oriented in a different direction than said first direction (D₁).

2. The absorbent article (20) of Claim 1 wherein said first region (56) is disposed along the proximal edge (52) of said side wrapping element (50) and said second region (58) is disposed laterally outboard of said first region (56), wherein said first region (56) has a first extensibility with a component that is primarily oriented in the transverse direction (T) and said second region (58) has a second extensibility with a component that is oriented more in the longitudinal direction (L) than the extensibility of said first region (56).

3. The absorbent article (20) of Claim 1 wherein said first region (56) comprises the transverse centerline T₁ of said side wrapping element (50) and said primary component of extensibility of said first region (56) is oriented primarily in the longitudinal direction (L) and said side wrapping element (50) comprises a plurality of second regions (58) located longitudinally outboard of said first region (56) on each side of said first region (56), and said second regions (58) have components of extensibility that angle away from the longitudinal centerline at angles that increase the farther the second regions (58) are spaced from the first region (56).

4. The absorbent article(20) of Claim 3 wherein said second regions (58) angle outwardly away from the longitudinal centerline (L) of the absorbent article (20).

5. The absorbent article (20) of Claim 3 wherein said second regions (58) angle inwardly away from the longitudinal centerline (L) of the absorbent article (20).

6. The absorbent article (20) of Claim 3 wherein said first and second regions (56, 58) comprise an uninterrupted continuum of extensibility.

7. The absorbent article (20) of Claim 3 wherein said first and second regions (56, 58) comprise discrete zones of extensibility.

8. The absorbent article (20) of Claim 7 wherein said first and second regions (56. 58) separated by less extensible regions of said side wrapping element (50).

9. The absorbent article (20) of Claim 3 wherein said first side wrapping element (50) has at least one generally longitudinally-oriented band of less extensibility therein.

10. The absorbent article (20) of Claim 9 wherein said band is curvilinear.

11. The absorbent article (20) of Claim 10 wherein said band is concave relative to the distal edge (54) of said side wrapping element (50).

12. The absorbent article (20) of Claim 10 wherein said band is convex relative to the distal edge (54) of said side wrapping element (50).

13. The absorbent article (20) of Claim 3 wherein at least some of lines drawn perpendicular to said components of extensibility of said side wrapping element (50) radiate outward from a single point.

14. The absorbent article (20) of Claim 2 wherein said side wrapping element comprises a staggered pattern of alternating first and second region (56, 58).

15. The absorbent article (20) of Claim 1 wherein at least a portion of said side wrapping element (50) is reinforced with a component that comprises a bubble contained within its structure.

16. The absorbent article (20) of Claim 1 wherein said side wrapping element (50) is integral with said main body portion (21).

17. The absorbent article (20) of Claim 1 wherein said side wrapping element (50) comprises a separate element joined to the garment-facing side (21B) of the main body portion (21) inboard of the longitudinal side edges (22) of said main body portion (21).

## Patentansprüche

1. Ein absorbierender Artikel (29) zum Tragen in einem Unterwäschestück eines Trägers, welches einen Schrittbereich mit einem Paar Seitenrändern umfaßt, wobei der genannte absorbierende Artikel (20) eine Längsdimension, welche sich in eIner Längsrichtung (L) erstreckt, und eine Querdimension, welche sich in einer Querrichtung (T) erstreckt, aufweist, wobei der genannte absorbierende Artikel (20) umfaßt:
einen Hauptkörperabschnitt (21), welcher einen absorbierenden Kern umfaßt, wobei der genannte Hauptkörperabschnitt (21) eine dem Körper zugewandte Seite (21A), eine der Kleidung zugewandte Seite (21B) und ein Paar Längsseitenränder (22) aufweist; und
ein Paar Seiten-umhüllende Elemente (50) zum Falten rund um die Seitenränder der Unterwäsche des Trägers, wobei die genannten Seiten-umhüllenden Elemente (50) mit den Längsseitenrändern (22) des genannten Hauptkörperabschnitts (21) verbunden sind und sich davon quer auswärts über die Längsseitenränder (22) des Hauptkörperabschnitts (21) hinaus zu distalen Rändern (54) erstrecken, dadurch gekennzeichnet, daß mindestens eines der genannten Seiten-umhüllenden Elemente (50) mit einem Muster von Bereichen von Verlängerbarkeit versehen ist, welche mindestens einen ersten Bereich (56), welcher eine Hauptkomponente von Verlängerbarkeit in einer ersten Richtung (D1) aufweist, und einen zweiten Bereich (58), welcher eine Hauptkomponente von Verlängerbarkeit in einer zweiten Richtung (D2) aufweist, welche in einer anderen Richtung als die genannte erste Richtung (D1) ausgerichtet ist, umfassen.

2. Der absorbierende Artikel (20) nach Anspruch 1, bei welchem der genannte erste Bereich (56) entlang dem proximalen Rand (52) des genannten Seiten-umhüllenden Elements (50) angeordnet ist und der genannte zweite Bereich (58) quer auswärts vom genannten ersten Bereich (56) angeordnet ist, wobei der genannte erste Bereich (56) eine erste Verlängerbarkeit mit einer Komponente aufweist, welche hauptsächlich in der Querrichtung (T) ausgerichtet ist, und der genannte zweite Bereich (58) eine zweite Verlängerbarkeit mit einer Komponente aufweist, welche mehr in der Längsrichtung (L) ausgerichtet ist als die Verlängerbarkeit des genannten ersten Bereichs (56).

3. Der absorbierende Artikel (20) nach Anspruch 1, bei welchem der genannte erste Bereich (56) die Quermittellinie (T) der genannten Seiten-umhüllenden Elemente (50) umfaßt und die genannte Hauptkomponente von Verlängerbarkeit des genannten ersten Bereichs (56) hauptsächlich in der Längsrichtung (L) ausgerichtet ist und das genannte Seiten-umhüllende Element (50) eine Mehrzahl von zweiten Bereichen (58) umfaßt, welche der Länge nach auswärts vom genannten ersten Bereich (56) an jeder Seite des genannten ersten Bereichs (56) angeordnet sind, und die genannten zweiten Bereiche (58) Komponenten von Verlängerbarkeit aufweisen, welche von der Längsmittellinie (L) weg winkelig verlaufen, in Winkeln, welche zunehmen, je weiter die zweiten Bereiche (58) vom ersten Bereich (56) beabstandet sind.

4. Der absorbierende Artikel (20) nach Anspruch 3, bei welchem die genannten zweiten Bereiche (58) von der Längsmittellinie (L) des absorbierenden Artikels (20) weg im Winkel nach außen verlaufen.

5. Der absorbierende Artikel (20) nach Anspruch 3, bei welchem die genannten zweiten Bereiche (58) von der Längsmittellinie (L) des absorbierenden Artikels (20) weg im Winkel einwärts verlaufen.

6. Der absorbierende Artikel (20) nach Anspruch 3, bei welchem die genannten ersten und zweiten Bereiche (56, 58) ein ununterbrochenes Kontinuum von Verlängerbarkeit umfassen.

7. Der absorbierende Artikel (20) nach Anspruch 3, bei welchem die genannten ersten und zweiten Bereiche (56, 58) diskrete Zonen von Verlängerbarkeit umfassen.

8. Der absorbierende Artikel (20) nach Anspruch 7, bei welchem die genannten ersten und zweiten Bereiche (56, 58) durch weniger verlängerbare Bereiche des genannten Seiten-umhüllenden Elements (50) getrennt sind.

9. Der absorbierende Artikel (20) nach Anspruch 3, bei welchem das genannte erste Seiten-umhüllende Element (50) mindestens ein allgemein der Länge nach ausgerichtetes Band von geringerer Verlängerbarkeit darin aufweist.

10. Der absorbierende Artikel (20) nach Anspruch 9, bei welchem das genannte Band gekrümmt ist.

11. Der absorbierende Artikel (20) nach Anspruch 10, bei welchem das genannte Band in bezug auf den distalen Rand (54) des genannten Seitenumhüllenden Elements (50) konkav ist.

12. Der absorbierende Artikel (20) nach Anspruch 10, bei welchem das genannte Band in bezug auf den distalen Rand (54) des genannten Seitenumhüllenden Elements (50) konvex ist.

13. Der absorbierende Artikel (20) nach Anspruch 3, bei welchem mindestens einige der Linien, welche lotrecht zu den genannten Komponenten von Verlängerbarkeit des genannten Seiten-umhüllenden Elements (50) gezogen sind, von einem einzigen Punkt auswärts strahlenförmig weggehen.

14. Der absorbierende Artikel (20) nach Anspruch 2, bei welchem das genannte Seiten-umhüllende Element ein gestaffeltes Muster von alternierenden ersten und zweiten Bereichen (56, 58) aufweist.

15. Der absorbierende Artikel (20) nach Anspruch 1, bei welchem mindestens ein Abschnitt des genannten Seiten-umhüllenden Elements (50) mit einem Bestandteil verstärkt ist, welcher eine Blase umfaßt, welche innerhalb seiner Struktur enthalten ist.

16. Der absorbierende Artikel (20) nach Anspruch 1, bei welchem das genannte Seiten-umhüllende Element (50) integral mit dem genannten Hauptkörperabschnitt (21) ist.

17. Der absorbierende Artikel (20) nach Anspruch 1, bei welchem das genannte Seiten-umhüllende Element (50) ein gesondertes Element umfaßt, welches mit der der Kleidung zugewandten Seite (21B) des Hauptkörperabschnitts (21) einwärts von den Längsseitenrändern (22) des genannten Hauptkörperabschnitts (21) verbunden ist.

## Revendications

1. Article absorbant (20) destiné à être porté dis le sous-vêtement d'un utilisateur, qui présente une partie d'entrejambe comportant une paire de bords latéraux, ledit article absorbant (20) présentant une dimension longitudinale s'étendit dis une direction longitudinale (L) et une dimension transversale s'étendant dans une direction transversale (T), ledit article absorbant (20) comprenant :
une partie de corps principal (21) comprenant une âme absorbante, ladite partie de corps principal (21) présentant un côté faisant face au corps (21A), un côté faisant face au sous-vêtement (21B), et une paire de bords longitudinaux latéraux (22); et
une paire d'éléments enveloppants latéraux (50) destinés à être pliés autour des bords latéraux du sous-vêtement de l'utilisateur, lesdits éléments enveloppants latéraux (50) étant réunis aux bords longitudinaux latéraux (22) de ladite partie de corps principal (21) et s'étendant latéralement vers l'extérieur au delà de ceux-ci, vers les bords distaux (54), caractérisé en ce qu'au moins un desdits éléments enveloppants latéraux (50) est pourvu d'un motif de régions d'extensibilité comprenant au moins une première région (56) qui a une composante principale d'extensibilité dans une première direction (D₁) et une deuxième région (58) qui a une composante principale d'extensibilité dans une deuxième direction (D₂) qui est orientée dans une direction différente de celle de ladite première direction (D₁).

2. Article absorbant (20) selon la revendication 1, dans lequel ladite première région (56) est placée le long du bord proximal (52) dudit élément enveloppant latéral (50), et ladite deuxième région (58) est placée latéralement à l'extérieur de ladite première région (56), dans lequel ladite première région (56) a une première extensibilité qui a une composante qui est d'abord orientée dans la direction transversale (T), et ladite deuxième région (58) a une deuxième extensibilité qui a une composante qui est plus orientée dans la direction longitudinale (L) que l'extensibilité de ladite première région (56).

3. Article absorbant (20) selon la revendication 1, dans lequel ladite première région (56) comprend la ligne médiane transversale (T₁) dudit élément enveloppant latéral (50), et ladite composante principale d'extensibilité de ladite première région (56) est orientée principalement dans la direction longitudinale (L), et ledit élément enveloppant latéral (50) comprend une pluralité de deuxièmes régions (58) situées longitudinalement à l'extérieur de ladite première région (56) sur chaque côté de ladite première région (56), et lesdites deuxièmes régions (58) ont des composantes d'extensibilité qui forment des angles à distance de la ligne médiane longitudinale (L) selon des angles qui augmentent d'autant plus que les deuxièmes régions (58) sont espacées de la première région (56).

4. Article absorbant (20) selon la revendication 3, dans lequel lesdites deuxièmes régions (58) forment des angles vers l'extérieur, à distance de la ligne médiane longitudinale (L) de l'article absorbant (20).

5. Article absorbant (20) selon la revendication 3, dans lequel lesdites deuxièmes régions (58) forment des angles vers l'intérieur, à distance de la ligne médiane longitudinale (L) de l'article absorbant (20).

6. Article absorbant (20) selon la revendication 3, dans lequel ladite première et lesdites deuxièmes régions (56, 58) comprennent un continuum ininterrompu d'extensibilité.

7. Article absorbant (20) selon la revendication 3, dans lequel ladite première et lesdites deuxièmes régions (56, 58) comprennent des zones discrètes d'extensibilité.

8. Article absorbant (20) selon la revendication 7, dans lequel ladite première et lesdites deuxièmes régions (56, 58) sont séparées par des régions moins extensibles dudit élément enveloppant latéral (50).

9. Article absorbant (20) selon la revendication 3, dans lequel ledit premier élément enveloppant latéral (50) comporte au moins une bande globalement orientée longitudinalement d'extensibilité moindre.

10. Article absorbant (20) selon la revendication 9, dans lequel ladite bande est curviligne.

11. Article absorbant (20) selon la revendication 10, dans lequel ladite bande est concave par rapport au bord distal (54) dudit élément enveloppant latéral (50).

12. Article absorbant (20) selon la revendication 10, dans lequel ladite bande est convexe par rapport au bord distal (54) dudit élément enveloppant latéral (50).

13. Article absorbant (20) selon la revendication 3, dans lequel au moins quelques-unes des lignes tirées perpendiculairement auxdites composantes d'extensibilité dudit élément enveloppant latéral (50) rayonnent vers l'extérieur à partir d'un point unique.

14. Article absorbant (20) selon la revendication 2, dans lequel ledit élément enveloppant latéral comprend un motif étagé de première et deuxièmes régions alternées (56, 58).

15. Article absorbant (20) selon la revendication 1, dans lequel au moins une partie dudit élément enveloppant latéral (50) est renforcée avec un composant qui comprend une bulle contenue à l'intérieur de sa structure.

16. Article absorbant (20) selon la revendication 1, dans lequel ledit élément enveloppant latéral (50) est solidaire de ladite partie de corps principal (21).

17. Article absorbant (20) selon la revendication 1, dans lequel ledit élément enveloppant latéral (50) comprend un élément séparé réuni au côté faisant face au sous-vêtement (21B) de la partie de corps principal (21) à l'intérieur des bords longitudinaux latéraux (22) de ladite partie de corps principal (21).
